## Europäisches Patentamt
## European Patent Office
## Office européen des brevets

(19)

(11) Numéro de publication: **0 068 931**
**A2**

(12)

# DEMANDE DE BREVET EUROPEEN

(21) Numéro de dépôt: **82400989.8**

(22) Date de dépôt: **27.05.82**

(51) Int. Cl.³: **A 61 B 6/00, A 61 B 6/02**

(30) Priorité: **10.06.81 BE 2059203**

(43) Date de publication de la demande: **05.01.83**
**Bulletin 83/1**

(84) Etats contractants désignés: **DE FR IT NL**

(71) Demandeur: **THOMSON-CSF, 173, Boulevard Haussmann, F-75379 Paris Cedex 08 (FR)**

(72) Inventeur: **Munch, Joseph, THOMSON-CSF SCPI 173, bld Haussmann, F-75379 Paris Cedex 08 (FR)**

(74) Mandataire: **Dubreuil, Annie et al, THOMSON-CSF SCPI 173, Bld Haussmann, F-75379 Paris Cedex 08 (FR)**

(54) **Dispositif d'accouplement d'un porte-tube Rontgen à un bâti.**

(57) L'invention concerne un dispositif d'accouplement d'un porte-tube Röntgen (1) à un bâti (20) d'un récepteur d'image (6) dans un tomographe, à savoir un dispositif (9-13) d'accouplement dans lequel il est accouplé entre le bâti (20) du récepteur d'images (6) et le porte-tube Röntgen (1) une bielle d'accouplement (8) pour déplacer synchroniquement le récepteur d'images (6) et le porte-tube Röntgen (1).

La bielle d'accouplement (8) comporte à une extrémité un accouplement non désaccouplable (7) et à l'autre extrémité un accouplement désaccouplable (9-13). Cette bielle (8) est déplaçable axialement pour réaliser l'opération d'accouplement ou de désaccouplement.

EP 0 068 931 A2

## DISPOSITIF D'ACCOUPLEMENT D'UN PORTE-TUBE
## RONTGEN A UN BATI

L'invention a pour objet un dispositif d'accouplement d'un porte-tube Röntgen à un bâti, d'un récepteur d'images dans un tomographe par exemple.

Comme le fait est bien connu dans des tomographes radiologiques déterminés, le porte-tube Röntgen déplaçable doit pouvoir être accouplé d'une manière désaccouplable au bâti déplaçable du récepteur d'images dans un tomographe, cela pour pouvoir obtenir un déplacement synchrone entre le porte-tube Röntgen et le bâti du récepteur d'images. Jusqu'ici, on a fait usage d'une bielle d'accouplement qui d'une part est fixée au bâti du récepteur d'images de manière à fonctionner comme une charnière et qui d'autre part est accouplée d'une manière désaccouplable à la charnière du porte-tube Röntgen. D'un autre côté, la bielle d'accouplement doit pouvoir être désaccouplée pour permettre le libre déplacement du porte-tube Röntgen pour d'autres techniques d'application.

Un dispositif d'accouplement connu est par exemple le dispositif d'accouplement en baïonnette, à savoir un dispositf dans lequel on effectue un mouvement de rotation à la main pour réunir l'une à l'autre les deux parties du dispositif d'accouplement, dont une partie fait partie du porte-tube Röntgen et dont l'autre partie est montée à la partie supérieure de la bielle d'accouplement.

Un inconvénient important de ce dispositif d'accouplement réside dans le fait que l'opérateur du tomographe doit chaque fois se rendre à l'arrière du tomographe pour accoupler la bielle d'accouplement au porte-tube Röntgen ou pour la désaccoupler, et que pour le faire, il doit encore effectuer une manoeuvre relativement compliquée.

Un dispositif d'accouplement selon l'invention, permet à un opérateur de réaliser l'accouplement et le désaccouplement d'un

2

appareil tel qu'un porte-tube Röntgen à un bâti d'un équipement, en restant pour cette opération toujours du même côté de cet équipement, avec un minimum d'opérations ; l'accouplement ainsi réalisé étant au moins aussi solide que ceux réalisés selon l'art antérieur et plus rapide grâce à une part d'automatisation.

Selon l'invention, un dispositif d'accouplement d'un porte-tube Röntgen à un bâti comportant une bielle d'accouplement accouplée entre le bâti et le porte-tube Röntgen pour déplacer synchroniquement le bâti et le porte-tube Röntgen, cette bielle d'accouplement ayant à une extrémité un accouplement désaccouplable, est caractérisé en ce que l'accouplement désaccouplable est un accouplement électromagnétique permanent.

A titre d'exemple sans caractère limitatif, il est présenté ci-après une description d'une forme d'exécution choisie de l'accouplement conforme à l'invention, permettant de mieux comprendre l'invention. Cette description est accompagnée de trois figures parmis lesquelles :

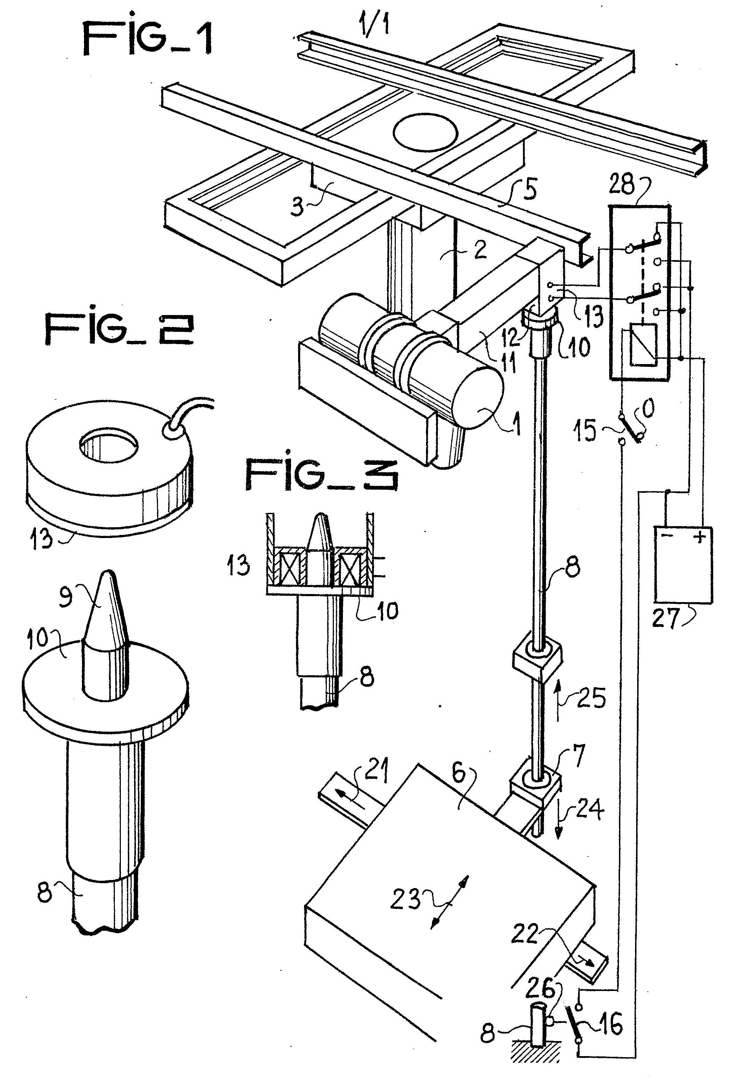

- la figure 1 est une vue en perspective d'une partie d'un tomographe ;

- la figure 2, une vue en perspective du dispositif d'accouplement, conforme à l'invention ;

- la figure 3, une coupe longitudinale de ce dispositif d'accouplement.

Dans ces figures, on remarque qu'un porte-tube Röntgen 1 d'un tomographe est suspendu à un télescope 2 et qu'ainsi le porte-tube Röntgen 1 est règlable en hauteur. Le télescope 2 est à son tour suspendu à un premier chariot 3 mobile dans un second chariot 4 lui-même mobile sur des rails 5 fixés au plafond, ce grâce à quoi le porte-tube Röntgen 1 peut se déplacer dans toutes les directions d'une manière bien connue. Au dessous est monté un bâti 20 déplaçable selon les flèches 21, 22, 23 avec le récepteur 6. A ce bâti, il est accouplé au moyen d'une masse sphérique 7 non désaccouplable, une bielle d'accouplement 8 axialement déplaçable dans cette masse, vers le bas selon la flèche 24, ou vers le haut selon la flèche 25. L'extrémité supérieure de cette bielle d'accouplement est équipée d'une goupille d'accouplement 9 se terminant en pointe et d'un disque de butée 10. Sur le bras 11 du porte-tube Röntgen 1, il est fixé un boîtier 12, monté transversalement de manière pivotante sur l'axe longitudinal de ce bras 11. Dans ce boîtier, il est monté un électro-aimant permanent 13 pourvu d'une ouverture centrale 14. L'extrémité inférieure 8A de la bielle d'accouplement 8, représentée en position basse, coopère avec des moyens mécaniques 26 classiques pour actionner dans cette position, un premier interrupteur 16.

Lorsque la bielle d'accouplement 8 doit être accouplée au porte-tube Röntgen 1, les chariots 3-4, doivent être déplacés et le télescope 2 manoeuvré de telle manière que l'électro-aimant permanent 13 soit en prise par l'intermédiaire de la goupille d'accouplement 9, et que le disque de butée 10 se trouve au repos tout contre l'électro-aimant permanent 13. En maintenant un second interrupteur 15, monté sur le tomographe, en position "ouvert" O pendant que l'on déplace la bielle d'accouplement 8 vers le haut, l'électro-aimant permanent 13 est exité par une tension continue de polarité telle que la goupille d'accouplement 9 est attirée par l'électro-aimant permanent 13 auquel elle est solidement fixée. Cette tension continue, fournie par des sorties (+,-) d'une source 27, passe par l'intermédiaire d'un relais 28 permettant d'inverser la polarité selon laquelle cette tension est appliquée à l'électro-aimant permanent 13 ; le relais 28 est actionné par l'interrupteur 16, à la condition que l'interrupteur 15 ne soit pas en position "ouvert" O, jusqu'à ce que l'extrémité 8A soit suffisamment relevée pour ne plus exercer sa commande. Par le fait que le dispositif d'accouplement est un accouplement électromagnérique permanent, la goupille d'accouplement 9 restera accouplée à l'électro-aimant 13, même en cas de panne de courant, ceci grâce à la force magnétique permanente d'accouplement. Pour désaccoupler l'accouplement, on met la bielle d'accouplement 8 en position verticale en déplaçant les chariots 3 et 4. On tire ensuite le télescope 2 vers le bas, ce grâce à

0068931

4

quoi la bielle d'accouplement 8 déplacée également vers le bas, selon le flèche 25 avec le télescope, actionne l'interrupteur 16 qui change automatiquement les polarités dans l'électro-aimant permanent 13, par la commande du relais 28. Grâce à cela, la bielle d'accouplement 8 est désaccouplée tout en restant dans sa position la plus basse. L'opérateur de l'appareil pousse le télescope 2 vers le haut de sorte que l'accouplement est parfaitement rompu.

Il va de soi que la forme et les dimensions de l'accouplement décrit ci-avant, ainsi que le montage des pièces de celui-ci dans leurs positions respectives peuvent différer, à condition de rester dans le cadre de l'invention.

5

## REVENDICATIONS

1. Dispositif d'accouplement d'un porte-tube Röntgen (1) à un bâti (20), comportant une bielle d'accouplement (8) accouplée entre le bâti (20) et le porte-tube Röntgen (1) pour déplacer synchroniquement le récepteur d'images (6) et le porte-tube Röntgen (1), cette bielle d'accouplement (8) ayant à une extrémité un accouplement désaccouplable (9-13), caractérisé en ce que l'accouplement désaccouplable (9-13) est un accouplement électromagnétique permanent.

2. Dispositif d'accouplement selon la revendication 1, caractérisé en ce que l'accouplement électromagnétique permanent (9-13) comporte une goupille d'accouplement (9) placée à l'extrémité supérieure de la bielle d'accouplement (8) et un électro-aimant permanent (13) placé dans un boîtier (12) qui à l'égard du porte-tube Röntgen (1) peut pivoter et où la goupille d'accouplement (9) en position accouplée est poussé et maintenue dans le trou central (14) de l'électro-aimant permanent (13) et y est maintenue.

3. Dispositif d'accouplement selon la revendication 2, caractérisé en ce que la goupille d'accouplement (9) se termine en pointe.

4. Dispositif d'accouplement selon la revendication 2, caractérisé en ce que la goupille d'accouplement (9) est pourvue d'un disque de butée (10) qui dans la position accouplée de la goupille d'accouplement (9) se trouve tout contre l'électro-aimant (13) qui forme l'autre partie de l'accouplement.

5. Dispositif d'accouplement selon la revendication 2, caractérisé en ce que des moyens (15-16) coopèrent avec la bielle d'accouplement (8) dans sa position la plus basse et verticale, pour commander l'accouplement électromagnétique permanent (13) et le désaccouplement de l'accouplement.

0068931

FIG_1

FIG_2

FIG_3